# EUROPEAN PATENT APPLICATION

(11) **EP 4 588 627 A1**
(43) Date of publication of application: **23.07.2025**
(21) Application number: 24382048.7
(22) Date of filing: 18.01.2024
(51) Int. Cl.: B25J 15/04, A61B 34/30, A61B 18/14

(54) **ELECTROSURGICAL DEVICE FOR COAGULATING BIOLOGICAL TISSUE**

(71) Applicant: Fundació Institut Hospital del Mar d'Investigacions Mèdiques, 08003 Barcelona (ES); Vecmedical Spain S.L., 08110 Montcada i Reixac (ES)
(72) Inventor: BURDIO PINILLA, Fernando, 08003 Barcelona (ES); IELPO, Benedetto, 08003 Barcelona (ES); SÁNCHEZ VELAZQUEZ, Patricia, 08003 Barcelona (ES); CARBÓ BECH, Albert, 08021 Barcelona (ES); CAMPS GONZALEZ, Ariadna, 08110 Montcada i Reixac (ES); ALONSO MERINO, Ángel, 08110 Montcada i Reixac (ES)
(74) Representative: ZBM Patents - Zea, Barlocci & Markvardsen

(57) **Abstract**

Electrosurgical device for coagulating biological tissue, the electrosurgical device comprises an electrode for coagulating biological tissue by radiofrequency, an electrically isolating member attached to the electrode, the electrosurgical device also comprising a tab to be held with a grasper, the tab comprising a body portion and a neck portion, the neck portion of the tab being attached to the electrically isolating member of the electrosurgical device, the neck portion of the tab connecting the electrically isolating member with the body portion of the tab.

## Description

The present disclosure relates to an electrosurgical device for coagulation of biological tissue.

### BACKGROUND ART

Surgical procedures require a safe method of obtaining minimally invasive operative haemostasis. Haemostasis can be obtained by the coagulation of biological tissue and vessels, for example by the contact of an electrode to the tissue to be coagulated.

In laparoscopic surgeries, surgical instruments are introduced in the body of the patient by cannulas. In this type of surgeries, the surgical instruments cannot be grabbed and manipulated by their distal end and have to be controlled by the user from a more distant, but more easily accessible location, e.g. from outside the patient's body. Electrosurgical handheld devices for coagulation comprising an elongated rigid shaft and an electrode at its distal end are used, which allows to transmit the movement made by the user from a distance to the electrode performing the coagulation.

A problem of known electrosurgical devices for coagulation is that the rigid shaft complicates the navigation and accessibility of the electrode to difficult areas of the body of the patient undergoing the surgical procedure.

### DESCRIPTION OF THE INVENTION

The present disclosure discloses an electrosurgical device for coagulation of biological tissue that can better navigate through the body during the surgical procedure and can reach desired areas to be treated.

In particular, the present disclosure relates to an electrosurgical device for coagulating biological tissue. The device comprises an electrode for coagulating biological tissue by radiofrequency, the electrode being activated by radiofrequency. The device comprises an electrically isolating member attached to the electrode. The device comprises a tab to be hold with a grasper. The tab comprises a body portion and a neck portion, the neck portion of the tab being attached to the electrically isolating member of the device. The neck portion of the tab connects the electrically isolating member with the body portion of the tab.

Since the neck portion of the tab is narrower than the body portion of the tab such that the body portion projects from the neck portion, the tab is suitable to be firmly and safely held, grasped, and manoeuvred by several kinds of laparoscopic and robotic graspers.

Surgical instruments such as graspers are able to pass through a cannula, e.g. for laparoscopic procedures: the provision on the device of a tab configured to be held by a grasper thus allows the device to be controlled from a point close to the tip or distal end of the electrode. A grasper from a robotic instrument can hold the electrode and control it, according to the movements of the surgeon that manipulates the robotic instrumentation from a console, to displace the electrode following a desired path.

Since the device is held and controlled from a point near the distal end of the electrode, there is no need for a long rigid shaft, as in known devices: proximally to the electrode, the device may be made substantially flexible, such that the rigid part of the device is significantly shorter than in the prior art, improving the ability of the device to navigate through the body and reach areas that cannot be reached with the known hand-operated device with a rigid shaft.

This grants the device with greater freedom of movement at its distal end than handheld devices in which the rigid shaft and its control from a distance impede having said freedom of movement at its distal end. The present device allows for a better and more precise control of the device, a broader range of mobility of the electrode of the device, and an overall improvement of the performance of the device. This device can be particularly used in laparoscopic procedures, in which the device can be introduced in the body of the patient through a cannula while a grasper can be introduced though another cannula and grab the device inside the body of the patient, thus having a flexible electrosurgical device and a control of the device by its distal end, near the electrode, which was not possible in other devices of the art.

In the context of this disclosure, it has to be understood that the device for coagulating biological tissue is not limited to the coagulation of biological tissues and that the device is also a device for coagulating other biological elements, such as biological vessels or any other element or part of the inner body. The device can also be referred to as a coagulation device.

In the context of this disclosure, it is considered as a grasper any robotic surgical instrument, laparoscopic instrument, and/or articulated instrument able to hold, grasp and/or control the device by the tab, including, but not limited to: a gripper, a stapler, a forceps, a laparoscopic grasper, instruments with force perception, etc.

In the context of this disclosure, the tab is defined as a projecting part or element of the device that protrudes or projects from the electrode, the electrically isolating element or a main part of the device, for example protruding in a perpendicular direction to the direction of the electrode and by which the device can be carried, held and manoeuvred.

In the context of this disclosure, a distal end or a distal part of the device, the electrode, the tab, the electrically isolating member, or any other element of the device, is defined as the part or end of said element that is closer to the tissue to be treated and more distant from the medical operator when carrying on a surgical process. In an electrode having a tubular body and a tip, the tip is the distal end of the electrode. A proximal end or proximal part of an element is defined as to the part or end of said element that is remote from the point to be treated and closer to the medical operator.

In an example, the electrosurgical device comprises a refrigeration channel to refrigerate the electrode, in an example to refrigerate an active portion of the electrode, in an example to refrigerate a distal part of the electrode. Coagulation of tissue without refrigeration have the problem that electrodes get stuck within the tissue and they get blocked and unable to be used. The refrigeration reduces the carbonization of the tissue while also enhances the coagulation (haemostasis) of the tissue. Therefore, it is advantageous that the device comprises both an electrode to coagulate the tissue and a refrigeration channel to refrigerate it, combined in the same device. Refrigeration of the electrode may be done by the flow of a refrigerant or cooling element into the refrigeration channel. This refrigerant can be any cooling liquid, cooling gas, or saline solution, in particular a liquid with low electrical conductivity.

In an example, the refrigeration channel is defined inside a lumen of the electrode. This refrigeration channel allows the refrigeration of the electrode by an inner circuit. Refrigeration by an inner hydraulic circuit generates a homogenic refrigeration, without the risk of the electrode being stuck within the tissue. Another advantage is that the refrigerant does not directly contact the tissue.

In an example, the device comprises an inlet refrigeration channel and an outlet refrigeration channel defined inside a lumen of the electrode and fluidically connected to one another, so that the cooling element enters the electrode from a refrigerant source, for example a bag of cooling liquid, by the inner refrigeration channel and leaves the electrode through the outlet refrigeration channel after refrigerating the electrode. In an example, the inlet refrigeration channel and the outlet refrigeration channel are connected to a hydraulic pump. In another example, the inlet refrigeration channel and the outlet refrigeration channel are connected to a common refrigerant source so that the refrigerant is directed into the electrode from the source by the inlet refrigeration channel and returned to the common refrigerant source through the outlet refrigeration channel, forming a closed-circuit refrigeration.

In an example, the electrosurgical device is a monopolar electrosurgical device, comprising only one electrode. The electrode is an electrode for coagulating tissue by radiofrequency, therefore being activated when radiofrequency is applied to the electrode. The device is a radiofrequency surgical device comprising a radiofrequency electrode. In an example, the electrode applies a radiofrequency to the biological tissue in a range between 100 kHz to 1 MHz, in an example between 250 kHz and 450 kHz, in an example between 300 kHz and 400 kHz, in an example 350 kHz.

In an example, the device comprises a switch for impeding the application of radiofrequency to the biological tissue by the electrode when a grasper is not in contact with said switch. The switch can be placed on the tab. The electrosurgical device comprises a switch having an open position and a closed position, the open position of the switch impeding the coagulation of biological tissue by radiofrequency and the closed position of the switch enabling the coagulation of biological tissue by radiofrequency. The switch allows blocking the application of radiofrequency to the tissue when a grasper is not properly in contact with the switch. Alternatively, the electrosurgical device comprises a sensor placed in the tab, the proximity of the grasper to the sensor enabling the application of radiofrequency.

The present disclosure also relates to an electrosurgical system for coagulation of biological tissue. The electrosurgical system comprises an electrosurgical device as described before and a source of cooling liquid.

### DESCRIPTION OF THE DRAWINGS

Non-limiting examples of the present disclosure will be described in the following, with reference to the appended drawings, in which:
Figure 1 shows a perspective view of an electrosurgical device according to a first embodiment of the present disclosure.
Figure 2 shows a perspective view of an electrosurgical device according to a first embodiment of the present disclosure, with the tab held by a robotic instrument.
Figures 3A, 3B and 3C shows a perspective view, a bottom view and an elevation view of the tab of the first embodiment of the disclosure.
Figure 4 shows a longitudinal section view of the device in Figure 1 in the X-Y plane.
Figure 5 shows a longitudinal section view of the device in Figure 1 in the X-Z plane.
Figure 6 shows an exploded view of the device.
Figure 7 shows a perspective view of a second embodiment of the disclosure.
Figure 8 shows a perspective view of a third embodiment of the disclosure, with the tab held by a robotic instrument.
Figure 9 shows a perspective view of a fourth embodiment of the disclosure, with the tab held by a robotic instrument.

### DETAILED DESCRIPTION

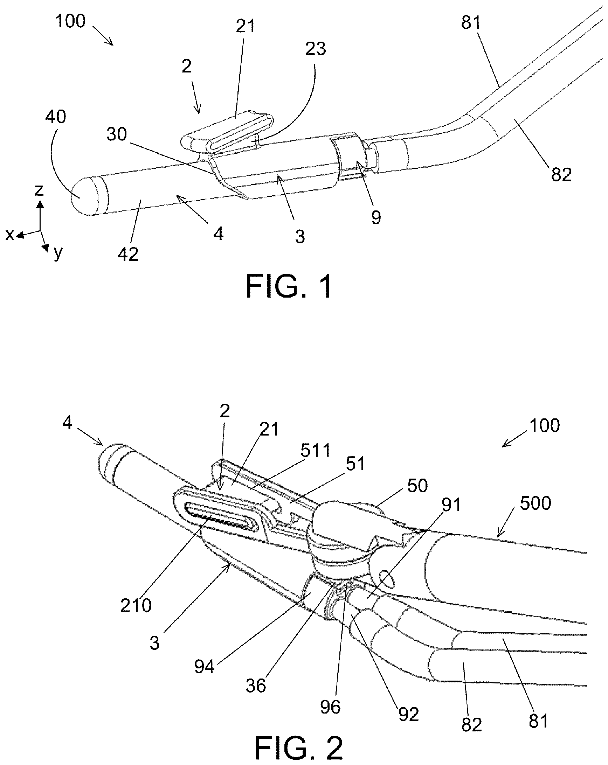

Figures 1 to 6 show a first embodiment of an electrosurgical device 100 for coagulating biological tissue.

Figures 1 and 2 show an electrosurgical device 100 for coagulating biological tissue. The device 100 comprises an electrode 4 for coagulating biological tissue, an electrically isolating member 3 attached to the electrode 4, and a tab 2 to be held with a grasper. Figure 1 shows the electrosurgical device 100, while Figure 2 shows the electrosurgical device 100 being grabbed by the tab 2 with a grasper. Contacting the electrode 4 to the biological tissue or vessel produces the coagulation of said tissue or vessel, obtaining haemostasis in said tissue or vessel.

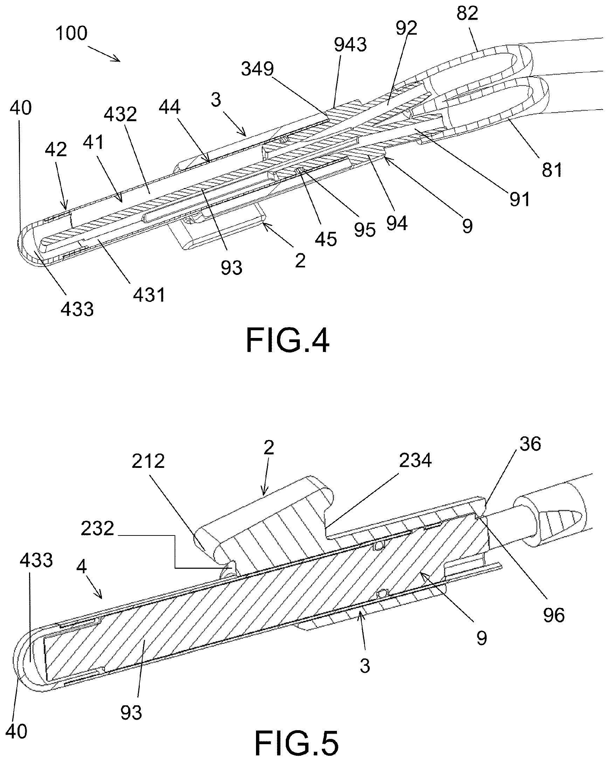

The electrode 4 shown is a tubular electrode, comprising a tubular, cylindric body, a semicircular distal end 40, and a lumen 41 defined by its inner wall. The electrode 4 is placed partially inside the electrically isolating member 3, such that an electrically isolated portion 44 of the electrode 4 (shown in Figure 4), and an active portion 42 of the electrode, corresponding to the protruding portion of the electrode 4, are defined. In the example shown, the electrode 4 is placed inside a through-hole 30 of the electrically isolating member 3. In alternative examples, the electrode 4 may be a planar electrode, or any other kind of electrode, and the electrode 4 may be placed inside the electrically isolating member 3 such that more than one electrically isolated portion 44 and/or active portion 42 of the electrode 4 are defined.

In an example, the active portion 42 of the electrode 4 protrudes between 15 and 25 mm from the electrically isolating member 3, in other examples protruding at least 15 mm, at least 10 mm, and at least 5 mm from the electrically isolating member 3. In an example, the distal end 40 of the electrode 4 is a semicircular tip having a radius of between 2 and 3 mm, in an example being 2,5 mm. For example, the tubular body of the electrode 4 may have a length of 35 mm and the distal end 40 of the electrode 4 has a length of 2,5 mm, the electrode 4 having a total length of 37,5 mm.

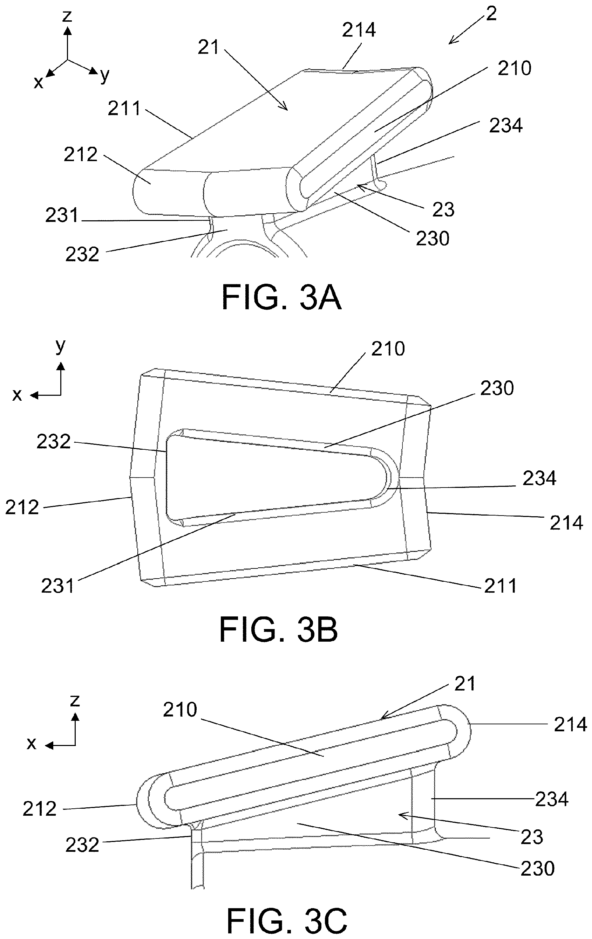

The tab 2 comprises a body portion 21 and a neck portion 23. The device 100 can be grasped, held, and manoeuvred by holding the body portion 21 of the tab 2 or the neck portion 23 of the tab 2. The tab 2 is shown being T-shaped, the body portion 21 having a prismatic head shape, or a generally parallelepipedic shape. In alternative embodiments, the tab 2 may be disc-shaped or have any other shape. The neck portion 23 of the tab 2 is attached to the electrically isolating member 3, connecting the electrically isolating member 3 with the body portion 21 of the tab 2. The tab 2 shown in the example is an integral part of the isolating member 3. In alternative embodiments, the tab 2 can be a separated, independent element attached to the isolating member 3. The neck portion 23 of the tab is placed at a distance as close as possible from the distal end 40 of the electrode 4 such that the movement of the device 100 is not hindered. In particular, the neck portion 23 of the tab 2 protrudes in the radial direction of the electrode 4, thus being elevated from the radial direction of the electrode 4, e.g. the neck portion 23 of the tab 2 protrudes between 2 and 3 mm from the electrode 4. This elevation allows for a better grabbing, holding and manoeuvring of the device by the tab 2. In alternate embodiments in which the electrode is not a tubular electrode, e.g. a planar electrode, the neck portion 23 protrudes perpendicularly to the longitudinal direction of the electrode. The neck portion 23 is higher in the radial direction of the electrode 4 at its proximal end than at its distal end, such that a distal end of the body portion 21 is inclined towards a distal end of the electrode 4, as shown in Figures 3A-3C.

In the example shown, the electrically isolating member 3 and the tab 2 are made of a semirigid material, such as plastic.

The device 100 also comprises a refrigeration channel 431, 432 (shown in Figures 4 and 5) to refrigerate the electrode 4 defined inside a lumen 41 of the electrode 4. In the example of Figure 1, the device 100 comprises an inlet refrigeration channel 431 and an outlet refrigeration channel 432 fluidically connected to a corresponding inlet refrigeration conduit 81 and outlet refrigeration conduit 82.

Figure 2 shows the electrosurgical device 100 held by the tab 2 with a grasper that is an articulated robotic surgical instrument 500 comprising an articulated joint 50 and two grips 51. This holding of the tab 2 by an articulated instrument allows for a better manipulation of the device 100. The tab 2 is shown grabbed at the body portion 21 of the tab 2, by the placement of the apertures 511 of the grips 51 of the instrument on longitudinal sides 210, 211 (shown in Figures 3A-3C) of the body portion 21 of the tab 2. The placement of the grips 51 may also contact the longitudinal sides 230, 231 of the neck portion 23 of the tab 2 (shown in Figures 3A-3C). Alternatively, the device 100 can also be grabbed by the neck portion 23 of the tab by a grasper.

Figures 3A, 3B and 3C show a perspective view, a bottom view and an elevation view of the tab 2 of the electrosurgical device 100.

As can be seen in Figure 3A, the body portion 21 of the tab 2 comprises a distal end 212, a proximal end 214 and two longitudinal sides 210, 211 on its sides. Analogously, the neck portion 23 of the tab 2 comprises a distal end 232, a proximal end 234 and two longitudinal sides 230, 231. The longitudinal sides 210, 211 of the body portion 21 of the tab 2 are bevelled. The bevelling allows for a better grasping and holding of the body portion 21 of the tab 2 by a grasper.

Figure 3B show that the body portion 21 of the tab 2 tapers in the longitudinal direction of the electrode 4 from a distal end 212 of the body portion towards a proximal end 214 of the body portion 21. The neck portion 23 of the tab 2 is also shown tapering in the longitudinal direction of the electrode 4 from a distal end 232 of the neck portion 23 towards a proximal end 234 of the neck portion 23. The tapering forms an opening angle between the longitudinal sides 210, 211, which may correspond to the angle of aperture of the grips 51 of an instrument 500, allowing for a better grasping, control, and holding of the device 100. Thus, the width of the body portion 21 and of the neck portion 23, defined as the length of the portions 21, 23 in the Y axis, increases from the proximal end 214, 234 towards the distal end 212, 232. Although the electrosurgical device 100 in the Figures is shown with a uniform tapering of both the neck portion 23 and the body portion 21 (the distance between each pair of longitudinal sides 210 - 211, 230 - 231 increasing from their respective proximal end 214, 234 towards their distal end 212, 232), the tapering can also be an ununiform tapering. Although in Figure 3B the distal end 212 of the body portion 21 is shown as an angled wall, said distal end 212 can alternatively have any known shape.

Figure 3B also shows that the body portion 21 of the tab 2 projects with respect to the neck portion 23 of the tab 2. In particular, the longitudinal sides 210, 211 of the body portion 21 of the tab 2 projects from the longitudinal sides 230, 231 of the neck portion 23 of the tab, and the distal end 212 and/or the proximal end 214 of the body portion 21 of the tab 2 projects from the corresponding distal end 232 or the proximal ends 234 of the neck portion 23 of the tab. This projection allows for a better grabbing of the device by the tab. This projection also eases the contact of a robotic surgical instrument placed on the longitudinal sides 210, 211 of the body portion 21 of the tab 2 with the corresponding longitudinal sides 230, 231 of the neck portion 23 of the tab 2.

Figure 3C shows that the neck portion 23 of the tab 2 is higher at its proximal end 234 than at its distal end 232, such that a distal end 212 of the body portion 21 is inclined towards a distal end 40 of the electrode 4. Thus, the proximal end 234 of the neck portion 23 protrudes more in the radial or perpendicular direction of the electrode 4 than its distal ends 232 thereof, defining an inclination angle. This inclination allows the user to control the movement of the device 100 more accurately.

Figures 4 and 5 show sectioned views of the electrode 4 of the device 100 in which the lumen 41 of the electrode 4 is shown in detail. Figure 4 shows a longitudinal sectioned view of the device in the X-Y plane, while Figure 5 shows a longitudinal sectioned view of the device in the X-Z plane, where the planes are the planes defined by the coordinate system shown in Figure 1.

Figure 4 show an inlet refrigeration channel 431 and an outlet refrigeration channel 432, said inlet and outlet refrigeration channels 431, 432 being defined inside a lumen 41 of the electrode 4. The inlet and outlet refrigeration channels are fluidically connected to one another, forming part of a refrigeration circuit. A cooling fluid, such as water, a saline solution or any other cooling fluid, is introduced to the inlet refrigeration channel 431, e.g. by a hydraulic pump and removed through the outlet refrigeration channel 432. As shown in Figure 4, the inlet and outlet refrigeration channels 431, 432 are fluidically connected with corresponding inlet and outlet refrigeration conduits 81, 82 at the proximal ends of the inlet and outlet refrigeration channels 431, 432. A first connecting channel 91 fluidically connects the inlet refrigeration conduit 81 with the inlet refrigeration channel 431 and a second connecting channel 92 fluidically connects the outlet refrigeration conduit 82 with the outlet refrigeration channel 432.The cooling fluid is pumped to the inlet refrigeration conduit 81, then into the inlet refrigeration channel 431 and returned to the pump through the outlet refrigeration channel 432 and the outlet refrigeration conduit 82 after refrigerating the electrode 4.

Figure 4 also show a partition wall 93 arranged along the lumen 41 of the electrode 4, the inlet and outlet refrigeration channels 431, 432 being further defined by the partition wall 93. Figure 4 also shows that the partition wall 93 defines a transmission area 433, the transmission area 433 being the area inside the lumen 41 of the electrode 4 in which the inlet and outlet refrigeration channels 431, 432 are fluidically connected to one another. In the example shown, the inlet and outlet refrigeration channels 431, 432 are fluidically connected to one another at a distal end 40 of the electrode 4. The device 100 shown in Figure 4 can also be defined as comprising a single refrigeration channel, the single refrigeration channel being U-shaped, defining an inlet refrigeration portion, an outlet refrigeration portion and a U-turn portion respectively corresponding to the inlet refrigeration channel 431, the outlet refrigeration channel 432 and the transmission area 433, such that a cooling fluid can be pumped into the refrigeration channel and pumped back from the same refrigeration channel. Alternatively, the channels 431, 432 can be fluidically connected in any known manner, such as by through-holes in the partition wall 93. In an alternate embodiment, the refrigeration channel may be a channel not defined by the lumen of the electrode, refrigerating the electrode from the outside of the electrode or being a conduit inserted into the electrode.

Figures 4 and 5 also show a connecting piece 9. The connecting piece 9 is attached to the lumen 41 of the electrode and to the electrically isolating member 3. In the example shown, the partition wall 93 is comprised in the connecting piece 9, extending longitudinally from a central portion 94 of the piece 9, the partition wall 93 being placed at the distal end of the central portion 94. The connecting piece 9 is placed attached to the electrode 4 of the device, such that the partition wall 93 of the connecting piece is placed inside the lumen 41 of the electrode. The first connecting channel 91 and the second connecting channel 92 are also comprised on the connecting piece 9. In particular, both connecting channels 91, 92 are placed in a proximal end of the central portion 94 of the connecting piece 9. In the example shown, the external diameter of the connecting channels 91, 92 are smaller than the inner diameter of the refrigeration conduits 81, 82 so that the connecting piece 9 is placed inside the refrigeration conduits 81, 82 securely tight. Alternatively, the inner diameter of the connecting channels 91, 92 are slightly larger than the external diameter of the refrigeration conduits 81, 82 so that the refrigeration channels are securely tight inside the connecting channels 91, 92.

The piece 9 also comprises fixing, sealing and/or attaching means for fixing, sealing and/or attaching the piece 9 to the electrode 4 and/or to the electrically isolating member 3. Figure 4 show an exemplary sealing means that comprise a recess 95 of the central portion 94 of the connecting piece 9 configured to receive an o-ring 45 of the lumen 41 of the electrode 4. In addition, the central portion 94 of the connecting piece 9 also comprise a protruded area 943 to perform dimensional interference with the electrically isolating member 3, the dimensional interference between the protruded area 943 and the isolating member 3 facilitating the placement and locking of the connecting piece 9 inside the lumen 41 of the electrode 4. Figure 4 shows the dimensional interference between the protruded area 943 of the connecting piece 9 and a proximal part 349 of the electrically isolating member 3, this part being shown as the proximal diameter of the through-hole 30 shown in Figure 1. Figure 5 show that the device 100 can also comprise clipping means, the clipping means comprising a recess 96 of the connecting piece and a protruding part 36 of the electrically isolating member 3.

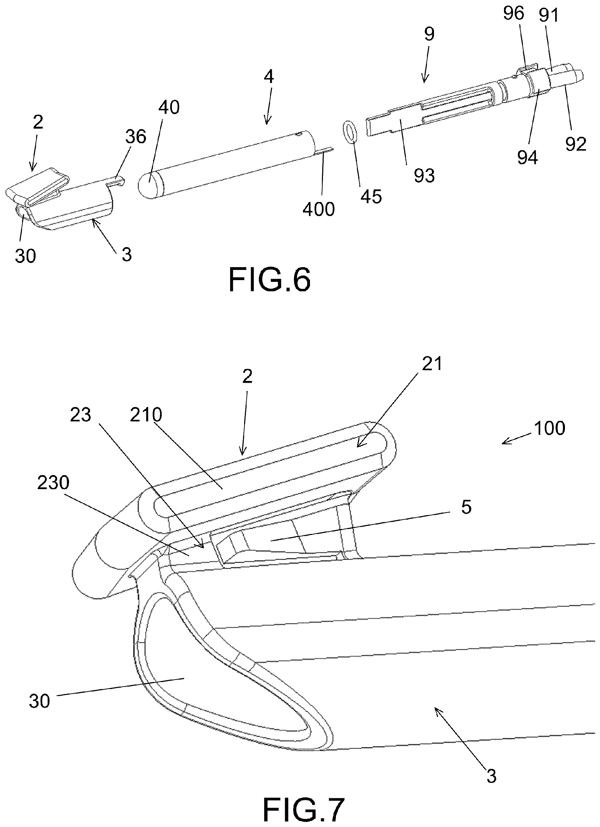

Figure 6 shows an exploded view of the electrosurgical device 100. In this figure, it can be seen the interlocking means between the protruding part 36 of the electrically isolating member 3 and the recess 96 of the connecting portion 94 of the connecting piece 4, as well as the through-hole 30 in which the electrode 4, and in particular the isolated portion 44 of the electrode 4, is placed. Figure 6 also shows a connection port 400 of the electrode 4 to connect the electrode 4 to a radiofrequency source.

Although the device 100 shown in the Figures comprises only one electrode 4, the device 100 being a monopolar electrosurgical device 100, the device can also be a bipolar device, comprising two electrodes, the distal end of both electrodes protruding from a single electrically isolating member. In that example, the refrigeration can be by a single refrigeration cable, or by a close-circuit refrigeration.

Figure 7 shows a second embodiment of the electrosurgical device disclosed herein. Analogous elements with other embodiments of the device have been referred with equivalent reference signs. The electrosurgical device 100 of the second embodiment further comprises a switch 5. The switch 5 is shown placed on the tab 2 of the device for impeding the application of radiofrequency and the activation of the electrode 4 when a grasper is not in contact with said switch 5. Radiofrequency can only be applied when the switch is in closed position, for example when a grasper is in contact with said switch. The contact of a grasper with the switch 5 closes the switch enabling the application of the radiofrequency. If the switch is in open position, for example when no grasper is in contact with the switch, the circulation of electricity through the switch and the application of radiofrequency to coagulate tissue is impeded. On a closed position, the surgeon or user may or may not activate the application of radiofrequency. The switch may alternatively have more than two positions.

The switch 5 is shown placed at a longitudinal side 231 of the neck portion 23 of the tab 2 so that a grasper placed on the neck portion 23 of the tab can contact the switch 5. In addition, the switch 5 is placed so that a grasper that is an articulated robotic surgical instrument 500 comprising two grips 51 with apertures 511 can grab the body portion 21 of the tab by placing the apertures 511 of the grips 51 on the longitudinal sides 210, 211 of the body portion 21 while contacting the switch 5 placed on a longitudinal side 230, 231 of the neck portion 23 of the tab 2. Alternatively, the device may comprise a switch on each longitudinal side 230, 231 of the tab 2, and/or the switch 5 may be alternatively placed on the body portion 21 of the tab 2. In alternate embodiments, the device may comprise a sensor connected to a controller, the controller enabling the application of radiofrequency to the electrode based on the information provided by the sensor. The sensor can be a pressure sensor, or an actuator, and be also connected to the controller via a cable or via Bluetooth.

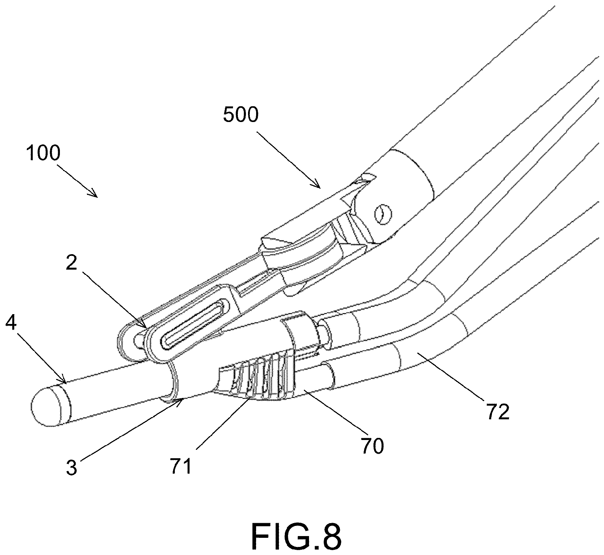

Figure 8 shows a third embodiment of the electrosurgical device disclosed herein. The electrosurgical device 100 is shown held by the tab 2 with an articulated robotic surgical instrument 500. Analogous elements with other embodiments of the device have been referred with equivalent reference signs. The device 100 of the third embodiment comprises a holding 70 for the placement therein of a suction tube, an irrigation tube, or an aspiration tube. This holding 70 is shown placed in a side of the isolating member 3 opposite to the tab 2, more particularly in an extension portion 71 of the electrically isolating member 3. The device of the third embodiment is shown as a coagulation-suction device, comprising a suction tube 72 placed on the holding 70. Alternatively, the holding 70 and the suction tube can be placed in a side of the isolating member next to the tab, or in any other known position, the scope of protection not being limited by the position of the holding or the suction tube. The extension portion 71 of the electrically isolating member 3 comprises grooves to prevent the suction of tissue parts that may block the suction tube 72. The suction tube 72 can be connected to a suction or an aspiration source such as a vacuum pump or any other suction source. the scope of protection not being limited by the suction source. In alternative embodiments, the device comprises an irrigation tube, e.g. for irrigating a cooling fluid, such as water or a saline solution.

The suction channel allows the device to clear the blood and fluid on the biological tissue, allowing for a better visualization of the area in which the surgical operation is performed. The removal of the blood and fluid also improves the coagulation of the tissue. The presence of a suction channel in the electrosurgical device for coagulation of the embodiment allows to perform the control of the bleeding and the coagulation of the tissue with the same instrument. In the context of this disclosure, it is defined as a suction channel any element that can be used primarily for removing obstructions, such as a suction channel, an aspiration channel, a blower, etc.

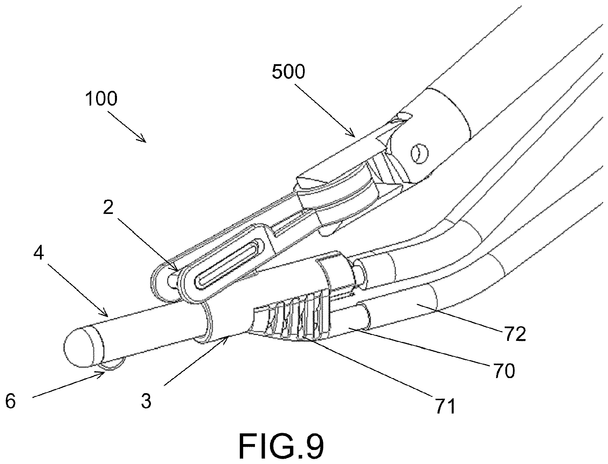

Figure 9 shows a fourth embodiment of the electrosurgical device disclosed herein. The electrosurgical device 100 is shown held by the tab 2 with an articulated robotic surgical instrument 500. Analogous elements with other embodiments of the device have been referred with equivalent reference signs. The device 100 of the fourth embodiment comprises a tissue cutter blade 6 to cut tissue. The cut of biological tissue can be performed by another means different than by a cutter placed in the device. For example, the cutting of the biological tissue can be performed by an external device different than the coagulation device and the user or surgeon can use two different devices to perform the coagulation and the cutting of the biological tissue. The blade 6 allows for the cutting of the biological tissue to be performed with the same electrosurgical device 100. In the example of Figure 8, the cutter blade 6 is shown placed in the active portion 42 of the electrode, in a side of the electrode 4 opposite from the tab 2.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples but should be determined only by a fair reading of the claims that follow. If reference signs related to drawings are placed in parentheses in a claim, they are solely for attempting to increase the intelligibility of the claim and shall not be construed as limiting the scope of the claim.

Clause 1. Electrosurgical device for coagulating biological tissue, the electrosurgical device comprising an electrode for coagulating biological tissue by radiofrequency, an electrically isolating member attached to the electrode, the electrosurgical device also comprising a tab to be held with a grasper, the tab comprising a body portion and a neck portion, the neck portion of the tab being attached to the electrically isolating member of the electrosurgical device, the neck portion of the tab connecting the electrically isolating member with the body portion of the tab.

Clause 2. Electrosurgical device, according to clause 1, wherein a width of the body portion of the tab tapers from a distal end of the body portion towards a proximal end of the body portion.

Clause 3. Electrosurgical device, according to any of clauses 1 or 2, wherein longitudinal sides of the body portion of the tab are bevelled.

Clause 4. Electrosurgical device, according to any of clauses 1 to 3, wherein a width of the neck portion of the tab tapers in the longitudinal direction of the electrode from a distal end of the neck portion towards a proximal end of the neck portion.

Clause 5. Electrosurgical device, according to any of clauses 1 to 4, wherein the neck portion of the tab is higher at its proximal end than at its distal end, such that a distal end of the body portion is inclined towards a distal end of the electrode.

Clause 6. Electrosurgical device, according to any of clauses 1 to 5, wherein the body portion of the tab projects with respect to the neck portion of the tab.

Clause 7. Electrosurgical device, according to any of clauses 1 to 6, wherein the longitudinal sides of the body portion of the tab project from the longitudinal sides of the neck portion of the tab.

Clause 8. Electrosurgical device, according to any of clauses 1 to 7, wherein the distal end or the proximal end of the body portion of the tab projects from the corresponding distal end or the proximal ends of the neck portion of the tab.

Clause 9. Electrosurgical device, according to any of clauses 1 to 8, wherein the tab is made of an electrically isolating material.

Clause 10. Electrosurgical device, according to any of clauses 1 to 9, wherein the tab is made of a semirigid material.

Clause 11. Electrosurgical device, according to any of clauses 1 to 10, wherein the tab is made of a plastic material.

Clause 12. Electrosurgical device, according to any of clauses 1 to 11, wherein the tab is an integral part of the electrically isolating member of the device.

Clause 13. Electrosurgical device, according to any of clauses 1 to 12, wherein the electrode is a tubular electrode.

Clause 14. Electrosurgical device, according to any of clauses 1 to 13, wherein the device is a monopolar electrosurgical device.

Clause 15. Electrosurgical device, according to any of clauses 1 to 14, wherein the electrode is placed partially inside the electrically isolating member, such that an active portion of the electrode and an electrically isolated portion of the electrode are defined, the active portion of the electrode protruding at least 5 mm from the electrically isolating member.

Clause 16. Electrosurgical device, according to clause 15, wherein the active portion of the electrode protrudes at least 10 mm from the electrically isolating member.

Clause 17. Electrosurgical device, according to any of clauses 15 or 16, wherein the active portion of the electrode protrudes between 15 and 25 mm from the electrically isolating member.

Clause 18. Electrosurgical device, according to any of clauses 1 to 17, wherein the electrode of the device coagulates biological tissue by applying radiofrequency in a range between 100 kHz and 1 MHz.

Clause 19. Electrosurgical device, according to any of clauses 1 to 18, wherein the electrode of the device coagulates biological tissue by applying radiofrequency in a range between 300 kHz and 400 kHz.

Clause 20. Electrosurgical device, according to any of clauses 1 to 17, wherein the device is a high frequency radiofrequency device, the electrode of the device being activated by high frequency radiofrequency.

Clause 21. Electrosurgical device, according to any of clauses 1 to 20, wherein the electrosurgical device comprises a refrigeration channel to refrigerate the electrode.

Clause 22. Electrosurgical device, according to any of clauses 1 to 21, wherein the electrosurgical device comprises a refrigeration channel to refrigerate an active portion of the electrode.

Clause 23. Electrosurgical device, according to any of clauses 21 or 22, wherein the refrigeration channel is defined inside a lumen of the electrode.

Clause 24. Electrosurgical device, according to any of clauses 21 to 23, wherein the device comprises an inlet refrigeration channel and an outlet refrigeration channel, said inlet refrigeration channel and outlet refrigeration channel being defined inside a lumen of the electrode, the inlet refrigeration channel and outlet refrigeration channel being fluidically connected to one another.

Clause 25. Electrosurgical device, according to clause 24, wherein the inlet refrigeration channel and outlet refrigeration channel are fluidically connected to one another at a distal end of the electrode.

Clause 26. Electrosurgical device, according to any of clauses 24 or 25, wherein the inlet refrigeration channel and the outlet refrigeration channel are further defined by a partition wall arranged along the lumen of the electrode.

Clause 27. Electrosurgical device, according to any of clauses 24 to 26, wherein the inlet refrigeration channel and the outlet refrigeration channel are fluidically connected with corresponding inlet refrigeration conduit and outlet refrigeration conduit at the proximal ends of the inlet refrigeration channel and outlet refrigeration channel.

Clause 28. Electrosurgical device, according to any of clauses 24 to 27, wherein the inlet refrigeration channel and the outlet refrigeration channel are connected to a hydraulic pump.

Clause 29. Electrosurgical device, according to any of clauses 24 to 28, wherein the inlet refrigeration channel and the outlet refrigeration channel are connected to a common refrigerant source, so that the refrigerant is directed into the electrode from the refrigerant source by the inlet refrigeration channel and returned to the common refrigerant source through the outlet refrigeration channel, forming a closed-circuit refrigeration.

Clause 30. Electrosurgical device, according to any of clauses 26 to 29, wherein the electrosurgical device comprises a connecting piece, the partition wall being defined in the connecting piece.

Clause 31. Electrosurgical device, according to clause 30, wherein the connecting piece is attached to the lumen of the electrode and to the electrically isolating member.

Clause 32. Electrosurgical device, according to any of clauses 30 or 31, wherein the device comprises sealing means between a recess of the connecting piece and a protrusion of the electrode.

Clause 33. Electrosurgical device, according to any of clauses 30 to 32, wherein the device comprises sealing means between a recess of the connecting piece and a protrusion of the electrically isolating member.

Clause 34. Electrosurgical device, according to clause 33, wherein the protrusion of the electrically isolating member is an o-ring.

Clause 35. Electrosurgical device, according to any of clauses 27 to 29, and any of clauses 29 to 33, wherein the connecting piece comprises a connecting channel for fluidically connecting the inlet and outlet refrigeration channels with the corresponding inlet and outlet refrigeration conduits.

Clause 36. Electrosurgical device, according to any of the previous clauses, wherein the electrosurgical device comprises a switch having an open position and a closed position, the open position of the switch impeding the activation of the electrode.

Clause 37. Electrosurgical device, according to clause 36, wherein the switch is in closed position when a grasper is in contact of the switch.

Clause 38. Electrosurgical device, according to clauses 36 or 37, wherein the switch is placed on the neck portion of the tab.

Clause 39. Electrosurgical device, according to any of clauses 36 to 38, wherein the switch is placed on the neck portion of the tab.

Clause 40. Electrosurgical device, according to any of the previous clauses, wherein the electrosurgical device comprises a sensor placed on the tab and a controller, the controller activating the application of radiofrequency by the electrode based on the information provided by the sensor.

Clause 41. Electrosurgical device, according to clause 40, wherein the sensor is a presence sensor.

Clause 42. Electrosurgical device, according to any of clauses 1 to 41, wherein the electrosurgical device comprises an aspiration tube.

Clause 43. Electrosurgical device, according to clause 42, wherein the electrosurgical device comprises an aspiration tube, the tube being placed in a holding for an aspiration tube.

Clause 44. Electrosurgical device, according to clause 43, wherein the holding for an aspiration tube is placed in a side of the isolating member opposite to the tab.

Clause 45. Electrosurgical device, according to any of clauses 1 to 44, wherein the electrosurgical device comprises a cutter blade to cut tissue, said cutter blade being placed in the active portion of the electrode.

Clause 46. Electrosurgical device, according to clause 45, wherein the cutter blade is placed in the active portion of the electrode in a side of the electrode opposite from the tab.

## Claims

1. Electrosurgical device for coagulating biological tissue, the electrosurgical device comprising an electrode for coagulating biological tissue by radiofrequency, an electrically isolating member attached to the electrode, the electrosurgical device also comprising a tab to be held with a grasper, the tab comprising a body portion and a neck portion, the neck portion of the tab being attached to the electrically isolating member of the electrosurgical device, the neck portion of the tab connecting the electrically isolating member with the body portion of the tab.

2. Electrosurgical device, according to claim 1, wherein a width of the body portion of the tab tapers from a distal end of the body portion towards a proximal end of the body portion.

3. Electrosurgical device, according to claim 1 or 2, wherein longitudinal sides of the body portion of the tab are bevelled.

4. Electrosurgical device, according to any of claims 1 to 3, wherein a width of the neck portion of the tab tapers from a distal end of the neck portion towards a proximal end of the neck portion.

5. Electrosurgical device, according to any of the previous claims, wherein the neck portion of the tab is higher at its proximal end than at its distal end, such that a distal end of the body portion is inclined towards a distal end of the electrode.

6. Electrosurgical device, according to any of the previous claims, wherein the electrosurgical device is a monopolar electrosurgical device.

7. Electrosurgical device, according to any of the previous claims, wherein the electrode is placed partially inside the electrically isolating member, such that an active portion of the electrode and an electrically isolated portion of the electrode are defined, the active portion of the electrode protruding at least 5 mm from the electrically isolating member.

8. Electrosurgical device, according to claim 7, wherein the active portion of the electrode protrudes between 15 and 25 mm from the electrically isolating member.

9. Electrosurgical device, according to any of the previous claims, wherein the electrode of the electrosurgical device coagulates biological tissue by applying radiofrequency in a range between 100 kHz and 1 MHz.

10. Electrosurgical device, according to any of the previous claims, wherein the electrosurgical device comprises a refrigeration channel to refrigerate the electrode.

11. Electrosurgical device, according to claim 10, wherein the electrosurgical device comprises an inlet refrigeration channel and an outlet refrigeration channel, said inlet refrigeration channel and outlet refrigeration channel being defined inside a lumen of the electrode, the inlet refrigeration channel and the outlet refrigeration channel being fluidically connected to one another.

12. Electrosurgical device, according to claim 11, wherein the inlet refrigeration channel and the outlet refrigeration channel are fluidically connected to one another at a distal end of the electrode.

13. Electrosurgical device, according to any of claims 11 or 12, wherein the inlet refrigeration channel and the outlet refrigeration channel are further defined by a partition wall arranged along the lumen of the electrode.

14. Electrosurgical device, according to claim 13, wherein the electrosurgical device comprises a connecting piece, the partition wall being defined in the connecting piece.

15. Electrosurgical device, according to any of the previous claims, wherein the electrosurgical device comprises a switch having an open position and a closed position, the open position of the switch impeding the activation of the electrode.
